(19) **Europäisches Patentamt / European Patent Office / Office européen des brevets**

(11) **EP 4 660 362 A1**

(12) **EUROPEAN PATENT APPLICATION**
published in accordance with Art. 153(4) EPC

(43) Date of publication:
**10.12.2025  Bulletin 2025/50**

(21) Application number: **24749973.4**

(22) Date of filing: **18.01.2024**

(51) International Patent Classification (IPC):
**D04H 3/011** (2012.01)       **A47L 13/16** (2006.01)
**A61F 7/03** (2006.01)        **A61F 13/15** (2006.01)
**B65D 65/46** (2006.01)       **D04H 3/16** (2006.01)

(52) Cooperative Patent Classification (CPC):
**A47L 13/16; A61F 7/03; A61F 13/15; B65D 65/46;
D04H 3/011; D04H 3/16**

(86) International application number:
**PCT/JP2024/001262**

(87) International publication number:
**WO 2024/162019 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.01.2023  JP 2023013089**

(71) Applicant: **Mitsui Chemicals Asahi Life Materials
Co., Ltd.
Tokyo 104-0028 (JP)**

(72) Inventors:
- **FUJIMOTO, Yuya
  Tokyo 100-0006 (JP)**
- **NAKANISHI, Yasuo
  Tokyo 100-0006 (JP)**

(74) Representative: **dompatent
Partnerschaft von
Patentanwälten und Rechtsanwälten mbB
Deichmannhaus am Dom
Bahnhofsvorplatz 1
50667 Köln (DE)**

(54) **BIODEGRADABLE NONWOVEN FABRIC AND USE OF SAME**

(57)    Provided is a biodegradable nonwoven fabric that is biodegradable and is extremely excellent in elasticity. This biodegradable nonwoven fabric is configured from fibers including a biodegradable thermoplastic resin, and is characterized in that a SS curve obtained by stretching in a 30°C environment does not have a yield point in the displacement 0-40 mm interval.

EP 4 660 362 A1

**Description**

FIELD

The present invention relates to a biodegradable nonwoven fabric and uses of the same.

BACKGROUND

Biodegradable nonwoven fabrics have conventionally been made from various biodegradable resins, including polylactic acid, and in recent years, with increasing awareness of sustainability promotion, they have been widely used in various fields and applications.

Patent Literature 1 below discloses a biodegradable nonwoven fabric comprising a side-by-side conjugated fiber formed by bonding two types of polymers together as a fiber assembly, which is soft and fluffy and which has practical elastic performance, wherein one of the polymers constituting the side-by-side conjugated fiber is polylactic acid and the other polymer is a copolymer of polyalkylene succinate and 1 to 6 mol% of lactic acid, and the side-by-side conjugated fiber comprises a polylactic acid-based latent crimp fiber having a latent crimp performance which brings about 30 spiral crimps/25 mm or more after dry heat treatment at 90°C.

[PTL 1] Japanese Unexamined Patent Publication (Kokai) No. 2010-270407

SUMMARY

[TECHNICAL PROBLEM]

However, the elasticity of the nonwoven fabric described in Patent Literature 1 is limited. Therefore, there is a need for a biodegradable nonwoven fabric having greater elasticity.

In view of the problems of the prior art described above, an object of the present invention is to provide a biodegradable nonwoven fabric that exhibits excellent elasticity while maintaining biodegradability.

[SOLUTION TO PROBLEM]

As a result of rigorous investigation and repeated experimentation in order to achieve the object described above, the inventors have unexpectedly discovered that the elasticity of a biodegradable nonwoven fabric which is composed of a fiber containing a biodegradable thermoplastic resin and in which an SS curve obtained by elongation in atmosphere at 30°C does not have a yield point in a displacement range of 0 to 40 mm is extremely improved, and thus, have completed the present invention.

Specifically, the present invention is as follows.

[1] A biodegradable nonwoven fabric composed of a fiber containing a biodegradable thermoplastic resin, wherein an SS curve obtained by elongation in an atmosphere at 30°C does not have a yield point in a displacement range of 0 mm to 40 mm.

[2] The biodegradable nonwoven fabric according to [1], wherein a machine direction 3% modulus index (N/30 mm/(g/m$^2$)) in a tensile test is greater than 0 and 0.225 or less.

[3] The biodegradable nonwoven fabric according to [2], wherein the machine direction 3% modulus index (N/30 mm/(g/m$^2$)) is 0.096 or less.

[4] The biodegradable nonwoven fabric according to any one of [1] to [3], wherein a proportion of a high mobility component as measured by pulse NMR at 30°C is 4.5 to 7.9%.

[5] The biodegradable nonwoven fabric according to any one of [1] to [4], wherein in polarized Raman spectroscopy of the fiber, a ratio I///I⊥ of a peak intensity I// at 1612 cm$^{-1}$ in a Raman spectrum as measured with polarized light parallel to a fiber axis to a peak intensity I⊥ at 1612 cm$^{-1}$ in a Raman spectrum as measured with polarized light perpendicular to the fiber axis is 3.2 to 7.9.

[6] The biodegradable nonwoven fabric according to any one of [1] to [5], wherein a glass transition temperature of the biodegradable thermoplastic resin is 25°C or lower.

[7] The biodegradable nonwoven fabric according to any one of [1] to [6], wherein the biodegradable thermoplastic resin is at least one selected from the group consisting of polybutylene succinate adipate, polybutylene adipate terephthalate, polyhydroxybutyrate valerate, and polyhydroxybutyrate butyrate.

[8] The biodegradable nonwoven fabric according to [7], wherein the biodegradable thermoplastic resin is polybutylene adipate terephthalate.

[9] The biodegradable nonwoven fabric according to any one of [1] to [8], wherein the biodegradable nonwoven fabric comprises at least one layer of a spunbond long-fiber nonwoven fabric.

[10] The biodegradable nonwoven fabric according to [8], wherein the biodegradable nonwoven fabric has a three-layer structure of a spunbond long-fiber nonwoven fabric/a meltblown microfiber nonwoven fabric/a spunbond long-fiber nonwoven fabric.

[11] A diaper, comprising the biodegradable nonwoven fabric according to any one of [1] to [10].

[12] A wipe, comprising the biodegradable nonwoven fabric according to any one of [1] to [10].

[13] A disposable warmer, comprising the biodegradable nonwoven fabric according to any one of [1] to [10].

[14] A lightweight packaging material, comprising the biodegradable nonwoven fabric according to any one of [1] to [10].

[ADVANTAGEOUS EFFECTS OF INVENTION]

The biodegradable nonwoven fabric of the present invention is biodegradable and exhibits excellent elasticity, and thus, can suitably be used for diapers, wipes, disposable warmers, lightweight packaging materials, and the like.

DESCRIPTION OF EMBODIMENTS

The embodiments of the present invention will be described in detail below.

The present embodiment provides a biodegradable nonwoven fabric composed of a fiber containing a biodegradable thermoplastic resin, wherein an SS curve obtained by elongation in an atmosphere at 30°C does not have a yield point in a displacement range of 0 mm to 40 mm.

In the biodegradable nonwoven fabric of the present embodiment, it is preferable that an SS curve obtained by elongation of the biodegradable nonwoven fabric at 30°C not have a yield point in a displacement range of 0 mm to 40 mm. Since a yield point is absent in a displacement range of 0 mm to 40 mm, the nonwoven fabric can elastically deform in response to tensile force in this displacement range, thereby exhibiting a very high elongation recovery rate when the force is released (i.e., exhibiting excellent elasticity). The method for determining the presence or absence of a yield point will be described later.

The biodegradable nonwoven fabric of the present embodiment preferably has an SS curve approximation in a displacement range of 0 mm to 40 mm of y=0.13x to y=1.38x, more preferably y=0.13x to y=0.63x, and further preferably y=0.13x to y=0.38x. If a yield point is present in a displacement range of 0 mm to 40 mm at 30°C and the SS curve approximation in the same range is y=0.13x or greater, the nonwoven fabric is likely to have excellent elasticity. The method of determining the SS curve approximation will be described later.

The biodegradable nonwoven fabric of the present embodiment is composed of a fiber containing a biodegradable thermoplastic resin. Examples of the biodegradable thermoplastic resin include polyhydroxybutyrate valerate, polyhydroxybutyrate butyrate, nylon 4, polyglycolic acid, polycaprolactone, polybutylene succinate, polybutylene succinate adipate, polybutylene terephthalate succinate, polybutylene succinate carbonate, polybutylene adipate terephthalate, polyethylene succinate, polyethylene terephthalate succinate, and polyvinyl alcohol. From the viewpoint of high biodegradability and elasticity, polyhydroxybutyrate valerate, polyhydroxybutyrate butyrate, and polybutylene adipate terephthalate are preferable. From the viewpoint of ease of achieving home compostability and soil compostability, polycaprolactone, nylon 4, polybutylene succinate adipate, and polybutylene adipate terephthalate are preferable, and from the viewpoint of ease of achieving marine compostability, polyhydroxybutyrate valerate, polycaprolactone, and nylon 4 are preferable. Details of home compostability, soil compostability, and marine compostability will be described later. From the viewpoint of ease of separation as a component of the final product, polycaprolactone and polybutylene succinate adipate, which can be melted by the heat of boiling water, are preferable.

The biodegradable nonwoven fabric of the present embodiment may have a laminated structure, for example, a laminated structure of SS, SMS, SMMS, SMSM, etc. "S" as used herein refers to a long-fiber nonwoven fabric by the spunbond method, and "M" referes to a microfiber nonwoven fabric by the meltblown method. Also, a short-fiber nonwoven fabric layer may be laminated on the biodegradable nonwoven fabric as a base material.

The shape of the fiber constituting the biodegradable nonwoven fabric of the present embodiment is not particularly limited, and may have a circular cross section or an irregular cross section such as flat, C-shaped, Y-shaped, or V-shaped cross section, and a circular cross section is preferable. Furthermore, the fiber may have an island-sea structure, a sheath-core structure, or a split fiber structure.

The fiber constituting the biodegradable nonwoven fabric of the present embodiment may further contain one or more kinds of another resin, a flame retardant, an inorganic filler, a softener, a plasticizer, a pigment, an antistatic agent, etc., in accordance with the purpose.

The fiber constituting the biodegradable nonwoven fabric of the present embodiment may contain a secondary component thermoplastic resin (hereinafter also referred to as a "secondary component resin") other than the biodegradable thermoplastic resin. The content of the secondary component resin is preferably 0.5% by mass or more and 50% by mass or less, more preferably 2% by mass to 50% by mass, further preferably 5% by mass to 30% by mass, and most preferably 5% by mass to 25% by mass, when the total mass of the nonwoven fabric is taken as 100% by mass. When the amount is 0.5% by mass or more, the crystallinity of the nonwoven fabric can be controlled, which facilitates control of the elasticity of the nonwoven fabric.

Examples of the secondary component resin include polyhydroxybutyrate valerate, polyhydroxybutyrate butyrate, nylon 4, polycaprolactone, polybutylene succinate, polybutylene succinate adipate, polybutylene terephthalate succinate, polybutylene succinate carbonate, polybutylene adipate terephthalate, polyethylene succinate, polyethylene terephthalate succinate, polyglycolic acid, polyvinyl alcohol, polypropylene, polyethylene terephthalate, and polyethylene. From the viewpoint of suitable biodegradability and elasticity, polyhydroxybutyrate valerate, polyhydroxybutyrate butyrate, and polybutylene adipate terephthalate are desirable, and from the viewpoint of ease of separation as a component of the final product, polycaprolactone and polybutylene succinate adipate, which can be melted by the heat of boiling water, are desirable. The secondary component resin may contain a non-biodegradable resin such as polypropylene, polyethylene terephthalate, and polyethylene, and from the viewpoint of satisfying the various biodegradability requirements described below, it is preferable that the content of the non-biodegradable resin be less than 10% by mass.

The biodegradable nonwoven fabric of the present embodiment may contain a surfactant in order to exhibit antistatic properties and water absorbency. Any of nonionic, anionic, or cationic surfactants can be used, and carboxylic acid, sulfonic acid, sulfate, phosphate, ester, ether, ester ether, alkanolamide, alkylamine, or quaternary ammonium can be used as the surfactant. Esters are preferable, and among the esters, sorbitan fatty acid esters, polyglycerin fatty acid esters, and polyoxyalkylene alkyl esters are particularly preferable. These surfactants may be used alone or in combination.

From the viewpoint of exhibiting sufficient antistatic properties and water absorbency, the adhesion rate of the surfactant is preferably 0.1 wt % or more and more preferably 2.0 wt % or less based on the mass of the nonwoven fabric. When the adhesion rate of the surfactant is 0.1 wt % or more and 2.0 wt % or less, sufficient performance can be obtained.

The surfactant can be applied by an existing method such as the kiss method, gravure method, spray method, etc., which can be appropriately selected according to the intended purpose. Specifically, when it is desired to exhibit the performance on only one side, a transfer method such as the kiss method or gravure method is preferable, and when it is desired to obtain the same performance on both sides, application by the spray method is preferable.

The basis weight of the biodegradable nonwoven fabric of the present embodiment is preferably 10 $g/m^2$ or more and 450 $g/m^2$ or less, more preferably 10 $g/m^2$ to 250 $g/m^2$, further preferably 12 $g/m^2$ to 70 $g/m^2$, and most preferably 12 $g/m^2$ to 40 $g/m^2$. When the basis weight is 10 $g/m^2$ or more, sufficient strength is obtained, and when it is 450 $g/m^2$ or less, sufficient elasticity can be maintained within the time required for general heat treatment such as calendaring.

The shear viscosity of the biodegradable nonwoven fabric of the present embodiment can be controlled by the molecular structure, molecular weight, and viscosity of the resin, the extrusion temperature, residence time, various additives including water, and the crystallinity and orientation of the nonwoven fabric during its production. For example, it is preferable to use polybutylene terephthalate adipate and polybutylene succinate adipate as the resin. When the nonwoven fabric is produced by the spunbond method, for example, the shear viscosity of the final nonwoven fabric is often about 1% to 30% lower than the shear viscosity of the raw material resin, depending on the thermal decomposition property of the resin, the water content of the resin, and the thermal history and the shear rate prior to extrusion.

The glass transition temperature of the biodegradable nonwoven fabric of the present embodiment is preferably 25°C or lower, more preferably 10°C or lower, and further preferably 0°C or lower. When the glass transition temperature is 25°C or lower, the mobility of the molecular chains is improved at room temperature, thus improving elasticity. In addition, since the SS curve obtained by elongation in an atmosphere at 30°C is less likely to have a yield point in a displacement range of 0 mm to 40 mm, elasticity is further improved. Furthermore, when the molecular mobility is high, even if embossing is performed at high temperature and high pressure in the thermal bonding step, defects such as pinholes are less likely to occur, and more elastic deformation behavior can be exhibited. From the viewpoint of setting the glass transition temperature in the range described above, the biodegradable nonwoven fabric of the present embodiment preferably contains a biodegradable thermoplastic resin such as polyhydroxybutyrate valerate, polyhydroxybutyrate butyrate, polybutylene adipate terephthalate, polycaprolactone, or polybutylene succinate adipate.

The biodegradable nonwoven fabric of the present embodiment has excellent elasticity as well as biodegradability, and can suitably be used in a wide range of fields, such as medical and sanitary materials, industrial materials, vehicle interior and exterior materials, soundproofing materials, sound absorbing materials, component transport trays, fruit and vegetable trays, food containers, agricultural materials such as seedling pods and mulch sheets, light packaging materials, and filter applications. It is particularly suitable for food filters such as coffee filters and tea bag filters, diapers, masks, agricultural materials, and lightweight packaging materials.

When the biodegradable nonwoven fabric of the present embodiment is composed of a biodegradable thermoplastic resin having a melting point of 100°C or lower, utilizing its property of melting and shrinking in hot water makes it easier to separate and recover the nonwoven fabric from a product that contains multiple materials, such as a diaper.

In the biodegradable nonwoven fabric of the present embodiment, the proportion of a high mobility component as measured by pulse NMR at 30°C is preferably 4.5% or more and 7.9% or less, more preferably 5.3% to 7.9%, and further preferably 5.7% to 7.9%. If the proportion of a high mobility component is 4.5% or more, the proportion of an amorphous mobility component at 30°C is high, and the number of free, unconstrained molecular chains increases, making the fabric less susceptible to plastic deformation under tension. A method for determining the proportion of a high mobility component as measured by pulse NMR at 30°C will be described later.

The machine direction 3% modulus index ($N/30\,mm/(g/m^2)$) of the biodegradable nonwoven fabric of the present embodiment is greater than 0, preferably 0.009 or more, and preferably 0.225 or less, more preferably 0.212 or less, further preferably 0.096 or less, and most preferably 0.080 or less. When the 3% modulus index is 0.225 or less, sufficient bending softness can easily be maintained. In particular, when the glass transition temperature is 25°C or lower, the effect of a 3% modulus index being greater than 0 and 0.212 or less is higher.

When the biodegradable nonwoven fabric of the present embodiment has an embossed portion and a non-embossed portion, the thickness index ($mm/(g/m^2)^{0.5}$) of the embossed portion, calculated by dividing the thickness of the embossed portion by the square root of the basis weight of the biodegradable nonwoven fabric, is preferably 2.84 or more and 7.78 or less, more preferably 5.60 or less, and further preferably 4.04 or less. When the thickness index of the embossed portion is 2.84 or more, the embossed portion is less likely to tear, and pinholes due to the process tension during processing of the nonwoven fabric are less likely to occur, and when it is 7.78 or less, sufficient bonding is achieved and the embossed portion is less likely to tear in response to tensile force such as process tension during processing of the nonwoven fabric. In particular, when the glass transition temperature is 25°C or lower, the effect of the thickness index of the embossed portion being 2.84 or more and 4.04 or less is higher. Generally, when the thickness of the embossed portion is excessively reduced, strength is significantly reduced, but if the glass transition temperature is 25°C or lower, the strength is less likely to decrease.

When a fiber constituting the biodegradable nonwoven fabric of the present embodiment is subjected to polarized Raman spectroscopy, the ratio I//I⊥ of the peak intensity I// at 1612 cm$^{-1}$ in a Raman spectrum of the fiber as measured with polarized light parallel to a fiber axis to the peak intensity I⊥ at 1612 cm$^{-1}$ in a Raman spectrum of the fiber as measured with polarized light perpendicular to the fiber axis is preferably 3.2 or more and 7.9 or less, more preferably 4.9 to 7.9, and further preferably 5.6 to 7.9. When I//I⊥ is 7.9 or less, excessive orientation is prevented, making it easy to reduce the thickness of the heat embossed portion. When I//I⊥ is 3.2 or more, the molecules constituting the fiber are sufficiently oriented, improving the stress against tension and thus improving the elasticity. A detailed method for measuring I//I⊥ will be described later.

The method for producing the biodegradable nonwoven fabric of the present embodiment is not limited, and a known method such as spunbonding, meltblowing, airlaid, carding, and papermaking can be used. The biodegradable nonwoven fabric of the present embodiment is preferably integrated by bonding, and examples of bonding methods that can be used include embossing, thermal bonding, columnar flow entanglement, mechanical entanglement, and needle punching. A long-fiber nonwoven fabric is preferable, as it can be produced efficiently and fuzzing after molding can be suppressed, and it is further preferable that the nonwoven fabric be produced by the spunbonding method.

When using the spunbonding method, a resin is heated, melted, and discharged from a spinneret, and the resulting spun filament is cooled using a known cooling device and drawn and attenuated using a suction device such as an air sucker. The filaments discharged from the suction device are then opened and deposited on a conveyor to form a web. The web formed on the conveyor is then partially thermally bonded using a partial thermal bonding device such as a heated embossing roll to obtain a spunbonded nonwoven fabric. The nonwoven fabric obtained by the spunbonding method has physical properties such as high fabric strength and an absence of short fiber dropout caused by damage to the bonded parts, and is low-cost and highly productive.

In the spunbonding method, a high-speed airflow drawing device using an air jet is generally employed, and the drawing force can be adjusted by the amount of air introduced into the drawing device. This drawing force (mN/m) was measured by inserting, into the drawing device, two fishing lines ("Ginscale (No. 2/natural/50 m roll)" nylon fishing line manufactured by Toray Industries, Inc.) each having a diameter of 0.235 mm and a length equal to the total length of the drawing device, measuring the tension with a spring scale connected to the fishing lines, and dividing the measured stress by the inserted length of the fishing lines. The drawing force is preferably 27 mN/m or more and 125 mN/m or less, more preferably 38 mN/m to 93 mN/m, and most preferably 70 mN/m to 93 mN/m. When the drawing force is 125 mN/m or less, filament breakage during spinning can adequately be prevented, and excessive increase in birefringence Δn after thermal bonding or fixed length heat treatment can be prevented by suppressing excessive orientation crystallization. When the drawing force is 27 mN/m or more, orientation crystallization can be sufficiently promoted and thermal bonding is enabled, resulting in a nonwoven fabric with sufficient strength.

The biodegradable nonwoven fabric of the present embodiment may have a three-layer structure of spunbond (S)/meltblown (M)/spunbond (S). The resins described above can be used as the materials constituting the meltblown layer.

The average fiber diameter of the meltblown layer is 0.3 μm or more and 7 μm or less, and preferably 0.6 μm or

more and 4 $\mu$m or less. When the fiber diameter is 0.3 $\mu$m or more, there is a tendency that spinning can be performed under mild conditions by the meltblown method and filament breakage during spinning can be suppressed. Conversely, when the average fiber diameter is 7 $\mu$m or less, there is a tendency that the meltblown layer penetrates into the gaps of the spunbond layer as fine fibers to fill the gaps and a dense structure is obtained.

The basis weight of the meltblown layer is preferably 1.0 g/m$^2$ or more and 100.0 g/m$^2$ or less, more preferably 1.0 g/m$^2$ or more and 60.0 g/m$^2$ or less, further preferably 1.2 g/m$^2$ or more and 16.0 g/m$^2$ or less, and most preferably 1.5 g/m$^2$ or more and 10.0 g/m$^2$ or less. When the basis weight of the meltblown layer is 1.0 g/m$^2$ or more, sufficient repair performance as a filter can be obtained. Conversely, when the basis weight of the meltblown layer is 100.0 g/m$^2$ or less, densification of the nonwoven fabric structure can be prevented, whereby an increase in pressure loss can be prevented.

The method of thermal bonding in the production of the biodegradable nonwoven fabric of the present embodiment is not limited, and may be thermal bonding using a combination of an embossing roll having a convex/concave surface pattern and a flat roll lacking convexities/concavities, or thermal bonding using a pair of embossing rolls both of which have a concave/convex surface pattern. The material of the roll surface is also not limited, and may be metal, rubber, resin, etc. When an embossing roll having a concave/convex surface pattern is used, thermal bonding can be performed at a roll temperature that is preferably 10°C or more lower than the melting point of the resin of the nonwoven fabric, at a linear pressure of preferably 5 N/mm or more and 100 N/mm or less, and more preferably 20 N/mm to 80 N/mm, and at a bonding area ratio of preferably 3% to 50%, and more preferably 6% to 40%. By performing thermal bonding within an appropriate range, both elasticity and bonding of the nonwoven fabric can be achieved. The "melting point of the resin of the nonwoven fabric" is measured by a method using a differential scanning calorimeter, which will be described later. When multiple melting peaks exist, the apex of the largest peak is taken as the melting point.

In the production of the biodegradable nonwoven fabric of the present embodiment, heat treatment at a fixed length may be performed regardless of whether the thermal bonding step is performed. The heat treatment at a fixed length is a heat treatment performed under a condition in which the dimensions of the nonwoven fabric in the length direction and width direction are fixed to remain unchanged during the heat treatment. Since a nonwoven fabric obtained by thermally bonding a nonwoven fabric web immediately after spinning and then performing the heat treatment at a fixed length has suitable surface smoothness and excellent stretchability in a thermal environment, tearing is unlikely to occur during molding processing and a molded product with a neat shape can easily be obtained with a neat shape. The heat treatment at a fixed length may be performed using a konwn method, such as hot air drying, pin tenter drying, hot plate, calendar processing, felt calendar processing, air-through processing, heat pressing, etc. The temperature range during the heat setting at a fixed length may be any temperature at which t the resin does not adhere to the equipment and the fibers of the nonwoven fabric are adequately bonded.

The biodegradable nonwoven fabric of the present embodiment can exhibit improved crystallinity and reduced thermal shrinkage under specific aging conditions. Specifically, the effects described above can easily be obtained by storing the fabric in an atmosphere of 40°C for 10 days or more.

EXAMPLES

The present invention is described in more detail below with reference to the Examples.

First, the measurement and evaluation methods used in the Examples and Comparative Examples will be described.

(1) Presence or Absence of Yield Point in Displacement Range of 0 mm to 40 mm in SS Curve Obtained by Elongation of Biodegradable Nonwoven Fabric at 30°C

Using an autograph AG-X plus (load cell: 1 kN) manufactured by Shimadzu Corporation, a 30 mm wide sample is elongated with the elongation origin set at 0.1% FS, a grip length of 100 mm, and a tensile rate of 300 mm/ min to obtain an SS curve. This elongation test is carried out 10 times, and the SS curve with a breaking stress closest to the average value of all 10 trials is selected. If the curve is convex upward (the SS curve lies above the straight line connecting the points at 0 mm and 40 mm), it is determined that the sample has a yield point.

(2) Approximation in Displacement Range of 0 mm to 40 mm in SS Curve Obtained by Elongation of Biodegradable Nonwoven Fabric at 30°C

The SS curve selected in (1) above is plotted as a graph up to 40 mm of displacement using Microsoft Excel. For this graph, the "Add Trendline" function of Excel is used, and "Linear" is selected so that a linear approximation is obtained.

(3) Basis Weight (g/m$^2$)

In accordance with JIS L-1913, a nonwoven fabric sample is cut so as to have a total area of 1500 cm$^2$ (3 sheets, 20 cm width $\times$ 25 cm length), and the basis weight is determined by converting the measured mass to a per unit area basis. In principle, the basis weight is measured by the above method, but if a sufficient sample size cannot be obtained, it may be measured using a sample of arbitrary size, such as 5 cm square.

(4) Average Fiber Diameter ($\mu$m)

A photograph at 500$\times$ magnification is taken using a VHX-700F microscope manufactured by Keyence Corporation, and the average value of 10 in-focus fibers within the observation field is determined.

(5) Glass Transition Temperature (°C)

Using a DSC6000 differential scanning calorimeter manufactured by PerkinElmer, Inc., approximately 5.0 mg of the nonwoven fabric is heated in a nitrogen atmosphere at a heating rate of 10°C/min from -50°C to a temperature near the melting point (Tm)+60°C, during which the secondary transition region corresponding to the glass-to-rubber transition is detected. The glass transition temperature (Tg) is defined as the onset temperature of the secondary transition .

(6) Melting Point (°C) of Resin of Nonwoven Fabric

Using a DSC6000 differential scanning calorimeter manufactured by PerkinElmer, Inc., 5.0 mg of the nonwoven fabric is heated in a nitrogen atmosphere from -50°C to 350°C at a heating rate of 10°C/min, during which the endothermic peak is detected. The endothermic peak is observed, and the apex of the peak is defined as the melting point. Note that multiple melting peaks may be observed depending on the type and number of resins used.

(7) Machine Direction 3% Modulus Index ((N/30 mm)/(g/m$^2$))

Using an autograph AG-X plus (load cell: 1 kN) manufactured by Shimadzu Corporation, a 30 mm wide sample is elongated with the elongation origin set at 0.1% FS, a grip length of 100 mm, and a tensile rate of 300 mm /min. The tensile force per 30 mm width at 3 mm elongation is defined as the 3% modulus. Ten measurements are made in the machine (longitudinal) direction of the nonwoven fabric, and the average value is calculated. The machine direction 3% modulus index is calculated by dividing the average value by the basis weight.

(8) Proportion (V, %) of High Mobility Component Measured by Pulse NMR at 30°C

The proportion (V, %) of the high mobility component obtained by pulse NMR can be calculated by the following method. A Minispec MQ20 manufactured by Bruker is used as the pulse NMR measuring device, and measurement is performed with 1H as the measurement nucleus, the solid echo method as the measurement method, and a number of accumulations of 256. Specifically, a glass tube with an outer diameter of 10 mm containing a measurement sample cut to a length of 1.0 cm is placed in a device maintained at 30 °C, and the T2 relaxation time of 1H is measured using the solid echo method 5 minutes after placement During the measurement, the repetition delay between scans is 5 times or more than the T1 relaxation time of the sample. From the magnetization decay curve (curve showing the change over time of magnetization intensity) obtained as described above, the signal intensity at 0.4 msec, when the signal intensity at the start of measurement at the start of acquisition is set to 100%, is defined as the proportion (V, %) of the high mobility component in the present embodiment.

(9) Ratio (I///I⊥) of Peak Intensity (I⊥) at 1612 cm$^{-1}$ in Raman Spectrum Measured with Polarized Light Parallel to Fiber Axis to Peak Intensity (I//) at 1612 cm$^{-1}$ in Raman Spectrum Measured with Polarized Light Perpendicular to Fiber Axis

Using a Raman spectrophotometer InViaReflex manufactured by Renishaw plc., the peak intensity (I//) at 1612 cm$^{-1}$ in the polarized Raman spectrum of a single fiber in the fiber layer (I) of the sample, which is placed so that the polarization plane of the excitation light is parallel to the fiber axis, is measured. Next, the sample is rotated by 90°, and the peak intensity (I⊥) at 1612 cm$^{-1}$ in a polarized Raman spectrum of the fiber of the sample, which is placed so that the polarization plane of the excitation light is perpendicular to the fiber axis, is measured. The average value of the ratio (I///I⊥) of (I//) to (I⊥) of 10 fibers is calculated. The larger the value of (I///I⊥), t the greater the molecular orientation along the fiber axis. The measurement is performed so that the optical axis of the analyzer of the polarized Raman spectrum is parallel to the optical axis of the excitation light.

The measurement conditions using the Raman spectrophotometer are as follows:

(Measurement Conditions)

Laser wavelength: 532 nm
Laser power at the measurement point: 1.5 mW
Objective lens: 20-fold (NA 0.40)
Diffraction grating: 1800 gr/mm
Exposure time: 2 seconds
Number of accumulations: 4

(10) Thickness Index $(mm/(g/m^2)^{0.5})$ of Embossed Portion

The nonwoven fabric sample is freeze-fractured in the cross-sectional direction, and the thickness of the embossed portion is observed using a scanning electron microscope (VE-8800) manufactured by Keyence Corporation. The magnification is 200-fold, and the thickness is measured at five points in total, and specifically, the center of the embossed portion, points 20 $\mu$m to either side of the center, and 20 $\mu$m further outward from each of those points. Of the five points, the maximum and minimum values are excluded, and the remaining three points are used as data for one embossment, and the average value of the thicknesses of the five embossments, i.e., a total of 15 points, is taken as the thickness (mm) of the embossed region. The thickness index is calculated by dividing this value by the square root of the basis weight of the nonwoven fabric.

(11) Biodegradability

The following four types of biodegradation tests are carried out, and if a fabric passes at least one of the tests, it is determined as biodegradable.

<Industrial Compostability>

In accordance with ISO 14855-1 (58±2°C) and JIS K 6953-1, a biodegradation test is performed at 58±2°C under conditions simulating those of an industrial composting plant, using organic components of municipal solid waste as compost. The degree of biodegradation is calculated as the ratio of the amount of carbon dioxide generated to the theoretical amount of carbon dioxide generated. If the degree of biodegradation reaches 90% or more within six months, the fabric is marked as "Pass (Good)"; otherwise, it is marked as "Fail (Poor)"..

<Home Compostability>

In accordance with ISO 14855-1 (28±2°C) and JIS K 6953-1, a biodegradation test is conducted at 28±2°C, and the degree of biodegradation is calculated as the ratio of the amount of carbon dioxide generated to the theoretical amount of carbon dioxide generated. When the degree of biodegradation reaches 90% or more within 6 months, the fabric is marked as pass (Good), and when it fails to do so, it is marked as fail (Poor).

<Soil Compostability>

In accordance with ISO 17556 (25±2°C), a biodegradation test is conducted at 25±2°C, and the degree of biodegradation is calculated as the ratio of the amount of carbon dioxide generated to the theoretical amount of carbon dioxide generated. When the degree of biodegradation reaches 90% or more within six months, the fabric is marked as pass (Good), and when it fails to do so, it is marked as fail (Poor).

<Marine Compostability>

In accordance with ASTM D6691 (30±1°C), a biodegradation test is performed at 30±1°C, and the degree of biodegradation is calculated as the ratio of the amount of carbon dioxide generated to the theoretical amount of carbon dioxide generated. When the degree of biodegradation reaches 90% or more within six months, the fabric is marked as pass (Good), and when it fails to do so, it is marked as fail (Poor).

(12) Elasticity (Elongation Recovery Rate) (%) of Nonwoven Fabric

Using an autograph AG-X plus (load cell: 1 kN) manufactured by Shimadzu Corporation, a 30 mm wide sample is elongated to a specified elongation of A% with the elongation origin set to 0.1% FS, a grip length of 100 mm, and a tensile rate of 300 mm/min. Before tension, reference marks are drawn to indicate a grip length of 100 mm, and after tension, the dimensions are measured again with a vernier caliper. The dimensional change before and after tension is expressed as B mm, and the elongation recovery rate is calculated using the following formula:

$$\text{Post-elongation shrinkage ratio (\%)} = B/(100+100A-100)$$

The closer the recovery rate is to 100%, the closer the sample length is to the length before tension, and thus, the higher the elasticity.

[Example 1]

Polybutylene adipate terephthalate (abbreviated as PBAT in the table) was melted and kneaded using a single screw extruder, extruded by the spunbond method at a discharge rate of 0.9 g/min/hole and a spinning temperature of 210°C, the filaments were drawn with a drawing force of 93 mN/m using a high-speed air flow drawing device and deposited on a moving collection surface to prepare a biodegradable long-fiber web (circular cross section).
Next, the web was thermally bonded using a pair of embossing rolls consisting of a roll having a convex/concave surface pattern on the surface thereof and a roll having a smooth surface, at a bonding area ratio of 11%, a temperature of 105°C for both rolls, and a roll linear pressure of 30 N/mm, to obtain a biodegradable nonwoven fabric having a basis weight of 20 g/m$^2$.

[Examples 2 to 7]

Biodegradable nonwoven fabrics were produced in the same manner as in Example 1, except that the line speed was adjusted to obtain the basis weights shown in the table below.

[Example 8]

A biodegradable nonwoven fabric was produced in the same manner as in Example 1, except that the embossing temperature was modified.

[Example 9]

Polybutylene succinate adipate (abbreviated as PBSA in the table) was melted and kneaded using a single screw extruder, and filaments were extruded onto a moving collection surface by the spunbonding method at a discharge rate of 0.9 g/min/hole, a spinning temperature of 140°C, and a drawing force of 93 mN/m to prepare a biodegradable long-fiber web (circular cross section). Next, the web was thermally bonded using a pair of embossing rolls, one having a convex/concave surface pattern and the other having a smooth surface pattern on the surface thereof and a roll having a smooth surface, at a bonding area ratio of 11%, a temperature of 75°C for both rolls, and a roll linear pressure of 30 N/mm, to obtain a biodegradable nonwoven fabric with a basis weight of 20 g/m$^2$.

[Example 10]

Polybutylene succinate (as shown in the table) was melted and kneaded using a single screw extruder, and filaments were extruded onto a moving collection surface by the spunbonding method at a discharge rate of 0.9 g/min/hole, a spinning temperature of 210°C, and a drawing force of 93 mN/m to prepare a biodegradable long-fiber web (circular cross section). Next, the web was thermally bonded using a pair of embossing rolls consisting of a roll having a convex/concave surface pattern on the surface thereof and a roll having a smooth surface, at a bonding area ratio of 11%, a temperature of 100°C for both rolls, and a roll linear pressure of 30 N/mm, to obtain a biodegradable nonwoven fabric with a basis weight of 20 g/m$^2$.

[Examples 11 and 12]

Biodegradable nonwoven fabrics were produced in the same manner as in Example 1, except that polybutylene succinate or polycaprolactone was added as a secondary component.

[Example 13]

A biodegradable nonwoven fabric was produced in the same manner as in Example 1, except that the drawing force was changed.

[Example 14]

Polybutylene adipate terephthalate raw fibers with a single fiber diameter of 30 $\mu$m were used as short fibers. These were passed through a carding machine to form a cross-laid nonwoven web having a basis weight of 20 g/m$^2$. The produced web was then placed on a 100-mesh wire net, and hydroentanglement treatment was performed using a high-pressure liquid flow treatment device with injection holes with a hole diameter of 0.08 mm arranged at a hole interval of 0.7 mm, to integrate the web. The liquid flow was injected once at a water pressure of 60 kg/cm$^2$ and once at a water pressure of 120 kg/cm$^2$. The liquid flow was then injected once at a water pressure of 120 kg/cm$^2$ from the reverse side. Thereafter, in order to remove excess moisture from the obtained web, it was subjected to drying treatment at 70°C using a hot air dryer to obtain a biodegradable nonwoven fabric.

[Example 15]

The biodegradable nonwoven fabric obtained in Example 14 was thermally bonded using a pair of embossing rolls, one of which had a concave/convex pattern on the surface thereof, with a bonding area ratio of 14%, a temperature of 105°C for both the upper and lower rolls, and a roll linear pressure of 30 N/mm, to obtain a biodegradable nonwoven fabric with a basis weight of 20 g/m$^2$.

[Example 16]

The biodegradable nonwoven fabric obtained in Example 14 was needle punched at a needle density of 400 needles/cm$^2$ to entangle the fiber web, to obtain a biodegradable nonwoven fabric having a basis weight of 20 g/m$^2$ was obtained.

[Comparative Example 1]

Polylactic acid (abbreviated as PLA in the table) was melted and kneaded using a single screw extruder, and filaments were extruded toward a moving collection surface by the spunbonding method at a discharge rate of 0.9 g/min/hole, a spinning temperature of 230°C, and a drawing force of 93 mN/m t to form a biodegradable long-fiber web (with a circular cross section). Next, the web was thermally bonded using a pair of embossing rolls consisting of a roll having a convex/concave surface pattern on the surface thereof and a roll having a smooth surface, at a bonding area ratio of 11%, a temperature of 130°C for both rolls, and a roll linear pressure of 20 N/mm, to obtain a biodegradable nonwoven fabric having a basis weight of 20 g/m$^2$.

[Comparative Example 2]

Polyethylene terephthalate (abbreviated as PET in the table) was melted and kneaded using a single screw extruder, and filaments were extruded toward a moving collection surface by the spunbonding method at a discharge rate of 0.9 g/min/hole, a spinning temperature of 290°C, and a drawing force of 93 mN/m to "form a biodegradable long-fiber web (with a circular cross section)". Next, the web was thermally bonded using a pair of embossing rolls consisting of a roll having a convex/concave surface pattern on the surface thereof and a roll having a smooth surface, at a bonding area ratio of 11%, a temperature of 220°C for both rolls, and a roll linear pressure of 20 N/mm, to obtain a biodegradable nonwoven fabric having a basis weight of 20 g/m$^2$.

[Comparative Example 3]

Polypropylene (abbreviated as PP in the table) was melted and kneaded using a single screw extruder, and filaments were extruded toward a moving collection surface by the spunbonding method at a discharge rate of 0.9 g/min/hole, a spinning temperature of 230°C, and a drawing force of 93 mN/m to form a biodegradable long-fiber web (circular cross section). Next, the web was thermally bonded using a pair of embossing rolls consisting of a roll having a convex/concave surface pattern on the surface thereof and a roll having a smooth surface, at a bonding area ratio of 11%, a temperature of 140°C for both rolls, and a roll linear pressure of 20 N/mm, to obtain a biodegradable nonwoven fabric having a basis weight of 20 g/m².

[Comparative Example 4]

Polycaprolactone (abbreviated as PCL in the table) was melted and kneaded using a single screw extruder, and filaments were extruded toward a moving collection surface by the spunbonding method at a discharge rate of 0.9 g/min/hole, a spinning temperature of 110°C, and a drawing force of 93 mN/m to prepare a biodegradable long-fiber web (circular cross section). Next, the web was thermally bonded using a pair of embossing rolls consisting of a roll having a convex/concave surface pattern on the surface thereof and a roll having a smooth surface, at a bonding area ratio of 11%, a temperature of 50°C for both rolls, and a roll linear pressure of 30 N/mm, to obtain a biodegradable nonwoven fabric having a basis weight of 20 g/m².

[Comparative Example 5]

A biodegradable nonwoven fabric was produced in the same manner as in Example 1, except that the embossing temperature was changed.

[Comparative Example 6]

A biodegradable nonwoven fabric was produced in the same manner as in Example 1, except that the drawing force was changed.

[Example 17]

PBAT was directly ejected from a meltblown nozzle onto a web (basis weight: 5.0 g/m²) prepared in the same manner as in Example 1 except that the line speed was adjusted so as to achieve a basis weight of 5.0 g/m², under the conditions of 1000 Nm³/hr at a spinning temperature of 210°C and a heated air temperature of 230°C to form a meltblown web (basis weight: 2.0 g/m², average fiber diameter: 2.1 μm). At this time, the distance from the meltblown nozzle to the spunbond web was set to 110 mm, and the suction wind speed at the collection surface directly below the meltblown nozzle was set to 7 m/sec. A spunbond web of PBAT that was the same as the above spunbond web was formed on the obtained meltblown web. The obtained laminated web was thermally bonded in the same manner as in Example 1 to obtain a nonwoven fabric having a total basis weight of 12.0 g/m².

[Example 18]

A biodegradable nonwoven fabric was produced in the same manner as in Example 18, except that for the first spunbond layer, the cross-sectional shape of the single fiber was sheath-core with a sheath-core ratio of 50/50 wt%, PBAT was used on the core side, and PBSA was used on the sheath side.

The physical properties and evaluation results of the nonwoven fabrics of Examples 1 to 18 and Comparative Examples 1 to 6 are shown in the following Tables 1 to 4. In the tables, in the SS curve obtained by elongation of the biodegradable nonwoven fabric at 70°C, the SS curve approximation in a displacement range of 0 to 40 mm is represented as "Condition A" when y=0.13x to y=1.38x, "Condition B" when y=0.13x to y=0.63x, and "Condition C" when y=0.13x to y=0.38x.

[Table 1]

| | | | Ex 1 | Ex 2 | Ex 3 | Ex 4 | Ex 5 | Ex 6 | Ex 7 | Ex 8 |
|---|---|---|---|---|---|---|---|---|---|---|
| Primary component resin | Type | - | PBAT | PBAT | PBAT | PBAT | PBAT | PBAT | PBAT | PBAT |
| | Content | mass% | 100 | 100 | 100 | 100 | 100 | 100 | 100 | 100 |
| | Glass transition temp (Tg) | °C | -30 | -30 | -30 | -30 | -30 | -30 | -30 | -30 |
| | Melting point (Tm) | °C | 120 | 120 | 120 | 120 | 120 | 120 | 120 | 120 |

(continued)

| | | | Ex 1 | Ex 2 | Ex 3 | Ex 4 | Ex 5 | Ex 6 | Ex 7 | Ex 8 |
|---|---|---|---|---|---|---|---|---|---|---|
| Secondary component re-sin | Type | - | - | - | - | - | - | - | - | - |
| | Content | mass% | - | - | - | - | - | - | - | - |
| | Glass transition temp (Tg) | °C | - | - | - | - | - | - | - | - |
| | Melting point (Tm) | °C | - | - | - | - | - | - | - | - |
| Drawing force | | mN/m | 93 | 93 | 93 | 93 | 93 | 93 | 93 | 93 |
| Embossing temp | | °C | 105 | 100 | 105 | 105 | 110 | 110 | 40 | 95 |
| Embossing pressure | | N/mm | 30 | 30 | 30 | 60 | 60 | 70 | 20 | 30 |
| Basis weight | | g/m² | 20 | 10 | 12 | 40 | 70 | 100 | 250 | 20 |
| Thickness | | mm | 0.10 | 0.05 | 0.06 | 0.16 | 0.22 | 0.31 | 0.62 | 0.10 |
| Machine direction 3% modulus | | N/30 mm | 0.23 | 0.14 | 0.26 | 1.91 | 6.74 | 21.20 | 56.30 | 0.23 |
| Machine direction 3% modulus index | | (N/30 mm)/(g/m²) | 0.012 | 0.014 | 0.022 | 0.048 | 0.096 | 0.212 | 0.225 | 0.012 |
| Embossed portion thickness | | mm | 0.0158 | 0.012 | 0.014 | 0.0182 | 0.0238 | 0.056 | 0.123 | 0.0182 |
| Embossed portion thickness index | | mm/(g/m²)$^{0.5}$ | 3.53 | 3.79 | 4.04 | 2.88 | 2.84 | 5.60 | 7.78 | 4.07 |
| Proportion of high mobility component measured by pulse NMR at 30°C | | % | 5.3 | 5.3 | 4.9 | 6.0 | 5.7 | 6.0 | 7.9 | 5.3 |
| I///I⊥ | | - | 7.9 | 7.9 | 7.9 | 7.9 | 7.9 | 7.9 | 7.9 | 7.8 |
| SS curve obtained by elongation at 30°C does not have yield point in 0 to 40 mm displacement range | | - | Good | Good | Good | Good | Good | Good | Good | Good |
| Linear approximation of SS curve obtained by elongation at 30°C satisfies condition A | | - | Good | Good | Good | Good | Good | Good | Poor | Good |
| Linear approximation of SS curve obtained by elongation at 30°C satisfies condition B | | - | Good | Good | Good | Good | Poor | Poor | Poor | Good |
| Linear approximation of SS curve obtained by elongation at 30°C satisfies condition C | | - | Good | Good | Good | Poor | Poor | Poor | Poor | Good |
| Biodegradability | Industrial | - | Good | Good | Good | Good | Good | Good | Good | Good |
| | Home | - | Good | Good | Good | Good | Good | Good | Good | Good |
| | Soil | - | Good | Good | Good | Good | Good | Good | Good | Good |
| | Marine | - | Poor | Poor | Poor | Poor | Poor | Poor | Poor | Poor |

(continued)

|  |  |  | Ex 1 | Ex 2 | Ex 3 | Ex 4 | Ex 5 | Ex 6 | Ex 7 | Ex 8 |
|---|---|---|---|---|---|---|---|---|---|---|
| Elasticity (elongation recovery rate) | 5% stretching | % | 90 | 82 | 84 | 97 | 100 | 100 | 100 | 90 |
| | 10% stretching | % | 92 | 81 | 85 | 100 | 100 | 100 | 100 | 92 |
| | 20% stretching | % | 91 | 81 | 83 | 93 | 94 | 100 | 100 | 91 |

[Table 2]

|  |  |  | Ex 9 | Ex 10 | Ex 11 | Ex 12 | Ex 13 | Ex 14 | Ex 15 | Ex 16 |
|---|---|---|---|---|---|---|---|---|---|---|
| Primary component resin | Type | - | PBSA | PBS | PBAT | PBAT | PBAT | PBAT | PBAT | PBAT |
| | Content | mass% | 100 | 100 | 80 | 80 | 100 | 100 | 100 | 100 |
| | Glass transition temp (Tg) | °C | -35 | -32 | -30 | -30 | -30 | -30 | -30 | -30 |
| | Melting point (Tm) | °C | 94 | 110 | 120 | 120 | 120 | 120 | 120 | 120 |
| Secondary component resin | Type | - | - | - | PBSA | PCL | - | - | - | - |
| | Content | mass% | - | - | 20 | 20 | - | - | - | - |
| | Glass transition temp (Tg) | °C | - | - | -35 | -60 | - | - | - | - |
| | Melting point (Tm) | °C | - | - | 94 | 60 | - | - | - | - |
| Drawing force | | mN/m | 93 | 93 | 93 | 93 | 84 | - | - | - |
| Embossing temp | | °C | 75 | 100 | 105 | 105 | 100 | - | 105 | - |
| Embossing pressure | | N/mm | 30 | 30 | 30 | 30 | 30 | - | 30 | - |
| Basis weight | | g/m$^2$ | 20 | 20 | 20 | 20 | 20 | 20 | 20 | 20 |
| Thickness | | mm | 0.10 | 0.12 | 0.10 | 0.10 | 0.10 | 0.10 | 0.10 | 0.10 |
| Machine direction 3% modulus | | N/30 mm | 2.15 | 4.30 | 0.21 | 0.22 | 0.20 | 0.18 | 0.25 | 0.18 |
| Machine direction 3% modulus index | | (N/30 mm)/(g/m$^2$) | 0.108 | 0.215 | 0.011 | 0.011 | 0.010 | 0.009 | 0.013 | 0.009 |
| Embossed portion thickness | | mm | 0.0158 | 0.0178 | 0.0148 | 0.0146 | 0.0152 | 0.1 | 0.0158 | 0.1 |
| Embossed portion thickness index | | mm/(g/m$^2$)$^{0.5}$ | 3.53 | 3.98 | 3.31 | 3.26 | 3.40 | 22.36 | 3.53 | 22.36 |
| Proportion of high mobility component measured by pulse NMR at 30°C | | % | 6.8 | 4.5 | 6.2 | 7.2 | 5.3 | 6.0 | 6.2 | 6.2 |
| I///I⊥ | | - | 5.6 | 5.8 | 7.2 | 6.9 | 4.2 | 7.4 | 7.6 | 7.2 |
| SS curve obtained by elongation at 30°C does not have yield point in 0 to 40 mm displacement range | | - | Good | Good | Good | Good | Good | Good | Good | Good |

(continued)

| | | | Ex 9 | Ex 10 | Ex 11 | Ex 12 | Ex 13 | Ex 14 | Ex 15 | Ex 16 |
|---|---|---|---|---|---|---|---|---|---|---|
| Linear approximation of SS curve obtained by elongation at 30°C satisfies condition A | | - | Good | Good | Good | Good | Poor | Good | Good | Good |
| Linear approximation of SS curve obtained by elongation at 30°C satisfies condition B | | - | Good | Good | Good | Good | Poor | Good | Good | Good |
| Linear approximation of SS curve obtained by elongation at 30°C satisfies condition C | | - | Good | Good | Good | Good | Poor | Good | Good | Good |
| Biodegradability | Industrial | - | Good | Good | Good | Good | Good | Good | Good | Good |
| | Home | - | Good | Poor | Good | Good | Good | Good | Good | Good |
| | Soil | - | Good | Poor | Good | Good | Good | Good | Good | Good |
| | Marine | - | Poor | Poor | Poor | Poor | Poor | Poor | Poor | Poor |
| Elasticity (elongation recovery rate) | 5% stretching | % | 85 | 81 | 89 | 85 | 75 | 81 | 91 | 80 |
| | 10% stretching | % | 82 | 80 | 86 | 84 | 74 | 80 | 90 | 79 |
| | 20% stretching | % | 81 | 78 | 87 | 82 | 70 | 80 | 91 | 76 |

[Table 3]

| | | | Comp Ex 1 | Comp Ex 2 | Comp Ex 2 | Comp Ex 3 | Comp Ex 4 | Comp Ex 5 | Comp Ex 6 |
|---|---|---|---|---|---|---|---|---|---|
| Primary component resin | Type | - | PLA | PET | PET | PP | PCL | PBAT | PBAT |
| | Content | mass% | 100 | 100 | 100 | 100 | 100 | 100 | 100 |
| | Glass transition temp (Tg) | °C | 56 | 70 | 70 | 0 | -60 | -30 | -30 |
| | Melting point (Tm) | °C | 165 | 250 | 250 | 160 | 60 | 120 | 120 |
| Secondary component resin | Type | - | - | - | - | - | - | - | - |
| | Content | mass% | - | - | - | - | - | - | - |
| | Glass transition temp (Tg) | °C | - | - | - | - | - | - | - |
| | Melting point (Tm) | °C | - | - | - | - | - | - | - |
| Drawing force | | mN/m | 73 | 73 | 73 | 73 | 93 | 93 | 73 |
| Embossing temp | | °C | 130 | 220 | 235 | 130 | 50 | 90 | 95 |
| Embossing pressure | | N/mm | 20 | 20 | 20 | 20 | 30 | 30 | 30 |
| Basis weight | | g/m$^2$ | 20 | 20 | 20 | 20 | 20 | 20 | 20 |
| Thickness | | mm | 0.16 | 0.17 | 0.15 | 0.16 | 0.09 | 0.10 | 0.10 |

(continued)

| | | | Comp Ex 1 | Comp Ex 2 | Comp Ex 2 | Comp Ex 3 | Comp Ex 4 | Comp Ex 5 | Comp Ex 6 |
|---|---|---|---|---|---|---|---|---|---|
| Machine direction 3% modulus | | N/30 mm | 16.30 | 28.00 | 15.00 | 16.00 | 2.13 | 0.23 | 0.19 |
| Machine direction 3% modulus index | | (N/30 mm)/(g/m²) | 0.815 | 1.400 | 0.750 | 0.800 | 0.107 | 0.012 | 0.010 |
| Embossed portion thickness | | mm | 0.021 | 0.0213 | 0.0159 | 0.0199 | 0.0158 | 0.0198 | 0.015 |
| Embossed portion thickness index | | mm/(g/m²)$^{0.5}$ | 4.70 | 4.76 | 3.56 | 4.45 | 3.53 | 4.43 | 3.35 |
| Proportion of high mobility component measured by pulse NMR at 30°C | | % | 0 | 0 | 0 | 0 | 7.9 | 5.3 | 5.3 |
| I///I⊥ | | - | 1.8 | 7.5 | 7.5 | 0.8 | 4.9 | 7.5 | 3.2 |
| SS curve obtained by elongation at 30°C does not have yield point in 0 to 40 mm displacement range | | - | Poor | Poor | Poor | Poor | Poor | Poor | Poor |
| Linear approximation of SS curve obtained by elongation at 30°C satisfies condition A | | - | Poor | Poor | Poor | Poor | (Good) | (Good) | (Good) |
| Linear approximation of SS curve obtained by elongation at 30°C satisfies condition B | | - | Poor | Poor | Poor | Poor | (Good) | (Good) | (Good) |
| Linear approximation of SS curve obtained by elongation at 30°C satisfies condition C | | - | Poor | Poor | Poor | Poor | (Good) | (Good) | (Good) |
| Biodegradability | Industrial | - | Good | Poor | Poor | Poor | Good | Poor | Good |
| | Home | - | Poor | Poor | Poor | Poor | Good | Poor | Good |
| | Soil | - | Poor | Poor | Poor | Poor | Good | Poor | Good |
| | Marine | - | Poor | Poor | Poor | Poor | Good | Poor | Poor |
| Elasticity (elongation recovery rate) | 5% stretching | % | 73 | 73 | Breakage | 72 | 52 | 72 | 54 |
| | 10% stretching | % | 71 | 70 | Breakage | 71 | 58 | 71 | 52 |
| | 20% stretching | % | 68 | 66 | Breakage | 64 | 51 | 64 | 50 |

[Table 4]

| | | | | | Ex 17 | Ex 18 |
|---|---|---|---|---|---|---|
| First layer | Primary component resin | Type | - | PBAT | PBAT |
| | | Content | mass% | 100 | 50 |
| | | Glass transition temp (Tg) | °C | -30 | -30 |
| | | Melting point (Tm) | °C | 120 | 120 |
| | Secondary component resin | Type | - | - | PBSA |
| | | Content | mass% | - | 50 |
| | | Glass transition temp (Tg) | °C | - | -35 |
| | | Melting point (Tm) | °C | - | 94 |
| | Basis weight | | g/m$^2$ | 5 | 5 |
| Second layer | Resin | Type | - | PBAT | PBAT |
| | | Content | mass% | 100 | 100 |
| | Fiber diameter | | μm | 2.1 | 2.1 |
| | Basis weight | | g/m$^2$ | 2 | 2 |
| Third layer | Primary component resin | Type | - | PBAT | PBAT |
| | | Content | mass% | 100 | 100 |
| | | Glass transition temp (Tg) | °C | -30 | -30 |
| | | Melting point (Tm) | °C | 120 | 120 |
| | Secondary component resin | Type | - | - | - |
| | | Content | mass% | - | - |
| | | Glass transition temp (Tg) | °C | - | - |
| | | Melting point (Tm) | °C | - | - |
| | Basis weight | | g/m$^2$ | 5 | 5 |
| Drawing force | | | mN/m | 93 | 93 |
| Embossing temp | | | °C | 105 | 105 |
| Embossing pressure | | | N/mm | 30 | 30 |
| Basis weight | | | g/m$^2$ | 12 | 12 |
| Thickness | | | mm | 0.06 | 0.07 |
| Machine direction 3% modulus | | | N/30 mm | 0.96 | 0.86 |
| Machine direction 3% modulus index | | | (N/30 mm)/(g/m$^2$) | 0.080 | 0.072 |
| Embossed portion thickness | | | mm | 0.014 | 0.013 |
| Embossed portion thickness index | | | mm/(g/m$^2$)$^{0.5}$ | 4.04 | 3.75 |
| Proportion of high mobility component at 30°C (pulse NMR) | | | % | 28 | 29 |
| I///I⊥ | | | - | 7.9 | 7.9 |
| SS curve obtained by elongation at 30°C does not have yield point in 0 to 40 mm displacement range | | | - | Good | Good |
| Linear approximation of SS curve obtained bv elongation at 30°C satisfies condition A | | | - | Good | Good |
| Linear approximation of SS curve obtained bv elongation at 30°C satisfies condition B | | | - | Good | Good |

(continued)

|  | | | Ex 17 | Ex 18 |
|---|---|---|---|---|
| Linear approximation of SS curve obtained bv elongation at 30°C satisfies condition C | | - | Good | Good |
| Biodegradability | Industrial | - | Good | Good |
| | Home | - | Good | Good |
| | Soil | - | Good | Good |
| | Marine | - | Poor | Poor |
| Elasticity (elongation recovery rate) | 5% stretching | % | 83 | 84 |
| | 10% stretching | % | 82 | 85 |
| | 20% stretching | % | 86 | 83 |

INDUSTRIAL APPLICABILITY

Since the biodegradable nonwoven fabric of the present invention has excellent elasticity as well as biodegradability, it can suitably be used in a wide range of fields, including medical and sanitary materials, industrial materials, vehicle interior and exterior materials, soundproofing materials, sound-absorbing materials, agricultural materials such as seedling pods and mulch sheets, light packaging materials, and filters.

**Claims**

1. A biodegradable nonwoven fabric composed of a fiber containing a biodegradable thermoplastic resin, wherein an SS curve obtained by elongation in an atmosphere at 30°C does not have a yield point in a displacement range of 0 mm to 40 mm.

2. The biodegradable nonwoven fabric according to claim 1, wherein a machine direction 3% modulus index (N/30 mm/(g/m$^2$)) in a tensile test is greater than 0 and 0.225 or less.

3. The biodegradable nonwoven fabric according to claim 2, wherein the machine direction 3% modulus index (N/30 mm/(g/m$^2$)) is 0.096 or less.

4. The biodegradable nonwoven fabric according to any one of claims 1 to 3, wherein a proportion of a high mobility component as measured by pulse NMR at 30°C is 4.5 to 7.9%.

5. The biodegradable nonwoven fabric according to any one of claims 1 to 3, wherein in polarized Raman spectroscopy of the fiber, a ratio I///I⊥ of a peak intensity I// at 1612 cm$^{-1}$ in a Raman spectrum as measured with polarized light parallel to a fiber axis to a peak intensity I⊥ at 1612 cm$^{-1}$ in a Raman spectrum as measured with polarized light perpendicular to the fiber axis is 3.2 to 7.9.

6. The biodegradable nonwoven fabric according to any one of claims 1 to 3, wherein a glass transition temperature of the biodegradable thermoplastic resin is 25°C or lower.

7. The biodegradable nonwoven fabric according to any one of claims 1 to 3, wherein the biodegradable thermoplastic resin is at least one selected from the group consisting of polybutylene succinate adipate, polybutylene adipate terephthalate, polyhydroxybutyrate valerate, and polyhydroxybutyrate butyrate.

8. The biodegradable nonwoven fabric according to claim 7, wherein the biodegradable thermoplastic resin is polybutylene adipate terephthalate.

9. The biodegradable nonwoven fabric according to any one of claims 1 to 8, wherein the biodegradable nonwoven fabric comprises at least one layer of a spunbond long-fiber nonwoven fabric.

10. The biodegradable nonwoven fabric according to claim 9, wherein the biodegradable nonwoven fabric has a three-layer structure of a spunbond long-fiber nonwoven fabric/a meltblown microfiber nonwoven fabric/a spunbond long-fiber nonwoven fabric.

11. A diaper, comprising the biodegradable nonwoven fabric according to any one of claims 1 to 3.

12. A wipe, comprising the biodegradable nonwoven fabric according to any one of claims 1 to 3.

13. A disposable warmer, comprising the biodegradable nonwoven fabric according to any one of claims 1 to 3.

14. A lightweight packaging material, comprising the biodegradable nonwoven fabric according to any one of claims 1 to 3.

## INTERNATIONAL SEARCH REPORT

| International application No. |
|---|
| **PCT/JP2024/001262** |

**A.  CLASSIFICATION OF SUBJECT MATTER**

*D04H 3/011*(2012.01)i; *A47L 13/16*(2006.01)i; *A61F 7/03*(2006.01)i; *A61F 13/15*(2006.01)i; *B65D 65/46*(2006.01)i; *D04H 3/16*(2006.01)i

FI:   D04H3/011; A47L13/16 A; A61F7/08 334B; A61F13/15 110; A61F13/15 120; B65D65/46; D04H3/16

According to International Patent Classification (IPC) or to both national classification and IPC

**B.  FIELDS SEARCHED**

Minimum documentation searched (classification system followed by classification symbols)

D04H3/011; A47L13/16; A61F7/03; A61F13/15; B65D65/46; D04H3/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus (JDreamIII)

**C.  DOCUMENTS CONSIDERED TO BE RELEVANT**

| Category* | Citation of document, with indication, where appropriate, of the relevant passages | Relevant to claim No. |
|---|---|---|
| A | WO 2022/176741 A1 (ASAHI KASEI KABUSHIKI KAISHA) 25 August 2022 (2022-08-25) comparative example 9 | 1-14 |
| P, X | WO 2023/032763 A1 (ASAHI KASEI KABUSHIKI KAISHA) 09 March 2023 (2023-03-09) examples 1, 24 | 1-14 |

☐ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **07 March 2024** | **19 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/001262**

| Patent document cited in search report | | | Publication date (day/month/year) | Patent family member(s) | | | Publication date (day/month/year) |
|---|---|---|---|---|---|---|---|
| WO | 2022/176741 | A1 | 25 August 2022 | EP | 4296417 | A1 | |
| | | | | comparative example 9 | | | |
| | | | | KR | 10-2023-0127321 | A | |
| | | | | CN | 116867938 | A | |
| | | | | CA | 3204737 | A | |
| | | | | TW | 202237924 | A | |
| WO | 2023/032763 | A1 | 09 March 2023 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010270407 A